# EUROPEAN PATENT SPECIFICATION

(11) **EP 4 536 158 B1**
(45) Date of publication and mention of the grant of the patent: **03.12.2025**
(21) Application number: 22741643.5
(22) Date of filing: 16.06.2022
(51) Int. Cl.: A61F 5/455

(54) **FEMALE EXTERNAL CATHETERS WITH APPLICATORS**
EXTERNE KATHETER FÜR FRAUEN MIT APLIKATOREN
CATHÉTERS EXTERNES FÉMININS AVEC APPLICATEURS

(43) Date of publication of application: 16.04.2025
(73) Proprietor: C. R. Bard, Inc., Franklin Lakes, NJ 07417 (US)
(72) Inventor: ROBICHAUD, Jonathan, Decatur, GA 30032 (US); WEISS, Rebecca, M., Atlanta, GA 30309 (US); JONES, Jill, Walthall, Avondale Estates, GA 30002 (US); CHENG, Jason, Jishen, Avondale Estates, GA 30002 (US)
(74) Representative: Hoffmann Eitle
(86) International application number: PCT/US2022/033886
(87) International publication number: WO 2023/244238

(56) References cited:
- WO-A1-2021/211729
- US-A1- 2009 182 296
- US-A1- 2009 254 040
- US-A1- 2016 317 698
- US-A1- 2016 374 848
- US-A1- 2022 152 352
- US-B1- 7 181 781

## Description

### BACKGROUND

Female external catheters ("FECs") are indicated for non-invasive urine-output management in incontinent female patients. Generally, the FECs operate by drawing urine away from the patients and into urine-drainage systems under vacuum. Intermittent FECs, which are typically used more frequently for shorter periods of time than some other FECs, are most often self-placed by the patients that use them. For this reason, such intermittent FECs should be intuitive and easy to place when needed for voiding urine.

Disclosed herein are applicators for FECs and methods thereof that address at least the foregoing.
WO 2021/211729 A1 relates to systems, devices, and methods utilizing means for securing a protruding portion of a fluid permeable body disposed between a wearer's labia in position during use, upon which the preamble of claim 1 is based.
US 2022/152352 A1 relates to a self-placeable external catheter that includes a catheter body having at least a top side and a bottom, a wicking area disposed on the top side, a finger covering extending from the bottom side of the catheter body creating a cavity configured to receive a user finger, and an aperture formed in the bottom side of the catheter, wherein the wicking area receives urine such that the urine passes through the wicking area, through the catheter body and through the aperture.

### SUMMARY

The present invention is defined by the female external catheter according to independent claim 1. Other preferred features are recited in the dependent claims. Disclosed herein is an FEC including, in some embodiments, a sidewall encircling a cavity that extends along a length of the FEC, a knoll of the sidewall that extends over the cavity in a sump end of the FEC, a catheter back opposite the cavity, and an applicator over the catheter back. The cavity is configured to open toward a patient for voiding urine into the cavity. The knoll is configured to be disposed over a vaginal introitus of the patient when the FEC is tucked between inner labia of the patient. The catheter back extends from the sump end of the FEC to a drainage end of the FEC opposite the sump end of the FEC. The applicator is configured for applying the FEC and holding the FEC in place during the voiding of the urine.

In some embodiments, the applicator is a finger pocket over the catheter back.

In some embodiments, the finger pocket includes a tubular body extending along at least a portion of the length of the FEC. The tubular body includes an open end opening toward the drainage end of the FEC and a closed end opposite the open end. The finger pocket is configured to accept a finger slidably disposed in the finger pocket from the open end of the finger pocket.

In some embodiments, the applicator is a finger clip over the catheter back.

In some embodiments, the finger clip includes a pair of arcuate clip arms extending along at least a portion of the length of the FEC. The pair of clip arms open toward each other to form a cradle configured to accept a finger slidably disposed in the finger clip from the drainage end of the FEC.

In some embodiments, each clip arm of the pair of clip arms includes a finger through hole therethrough providing a pair or finger through holes. The pair of finger through holes is configured to accept a finger and a thumb therein for pinching and holding the pair of arcuate clip arms.

In some embodiments, the applicator resembles a "T"-shaped handle over the catheter back.

In some embodiments, the handle includes a post supporting a crossbar that extends along at least a portion of the length of the FEC or orthogonally to the length of the FEC. The handle is configured to accept a pair of fingers slidably disposed under the crossbar for holding the handle.

In some embodiments, the applicator is a pinchable ridge over the catheter back.

In some embodiments, the ridge extends along at least a portion of the length of the FEC or orthogonally to the length of the FEC. The ridge is configured to accept a finger and a thumb over the ridge for pinching and holding the ridge.

In some embodiments, the applicator is a pinchable pad over the catheter back.

In some embodiments, the pad extends along at least a portion of the length of the FEC. The pad resembles a raised letter or shape configured to accept a finger and a thumb over the pad in either orientation of two orthogonal orientations for pinching and holding the pad.

In some embodiments, the raised letter or shape of the pad is selected from a raised "X," "H," "I," rectangle, square, and astroid.

In some embodiments, the applicator is an extendable button over the catheter back.

In some embodiments, the button has a retracted state and an extended state. The button in the retracted state is recessed into or lies against the catheter back. The button in the extended state is configured to accept a pair of fingers slidably disposed under the button for holding the button.

In some embodiments, the sump end of the FEC includes a sump in the cavity. The sump is configured to collect the urine during the voiding of the urine for withdrawal from the FEC.

In some embodiments, the catheter back includes a conduit extending along the length of the FEC from the sump through the drainage end of the FEC.

In some embodiments, the FEC further includes catheter tubing disposed in a portion of the conduit up to an entirety of the conduit.

In some embodiments, the sidewall and the catheter back form an integral body of the FEC, optionally, together with the applicator.

Also disclosed herein as an example not part of the claimed invention is a method of an FEC including, in some embodiments, a catheter-connecting step, a catheter-applying step, a pump-switching step, and a urine-voiding step. The catheter-connecting step includes connecting catheter tubing of the FEC to urine-drainage tubing of a remainder of a urine-drainage system. The FEC includes a knoll of a sidewall that encircles a cavity extending along a length of the FEC. The catheter-applying step includes applying the FEC such that the knoll is disposed over a vaginal introitus when the FEC is tucked between inner labia. The applying of the FEC utilizes an applicator over a catheter back of the FEC opposite the cavity. The pump-switching step includes switching on a pump of a pump unit of the urine-drainage system to draw a vacuum through the cavity. The urine-voiding step includes voiding urine into the FEC such that the urine is drawn into the cavity, through a sump in a sump end of the FEC, and out a drainage end of the FEC opposite the sump end of the FEC by way of the catheter tubing.

In some embodiments, the catheter-applying step includes inserting a finger into a finger pocket as the applicator.

In some embodiments, the finger pocket includes a tubular body extending along at least a portion of the length of the FEC. The tubular body includes an open end opening toward the drainage end of the FEC and a closed end opposite the open end. The finger pocket is configured to accept the finger in the finger pocket from the open end of the finger pocket.

In some embodiments, the catheter-applying step includes pinching or inserting a finger into a finger clip as the applicator.

In some embodiments, the finger clip includes a pair of arcuate clip arms extending along at least a portion of the length of the FEC. The pair of clip arms open toward each other to form a cradle configured to accept the finger in the finger clip from the drainage end of the FEC.

In some embodiments, each clip arm of the pair of clip arms includes a finger through hole therethrough providing a pair or finger through holes. The pair of finger through holes is configured to accept the finger and a thumb therein for the pinching of the finger clip.

In some embodiments, the catheter-applying step includes holding a "T"-shaped handle as the applicator.

In some embodiments, the handle includes a post supporting a crossbar that extends along at least a portion of the length of the FEC or orthogonally to the length of the FEC. The handle is configured to accept a pair of fingers slidably disposed under the crossbar for the holding of the handle.

In some embodiments, the catheter-applying step includes pinching a pinchable ridge as the applicator.

In some embodiments, the ridge extends along at least a portion of the length of the FEC or orthogonally to the length of the FEC. The ridge is configured to accept a finger and a thumb over the ridge for the pinching of the ridge.

In some embodiments, the catheter-applying step includes pinching a pinchable pad as the applicator.

In some embodiments, the pad extends along at least a portion of the length of the FEC. The pad resembles a raised letter or shape configured to accept a finger and a thumb over the pad in either orientation of two orthogonal orientations for the pinching of the pad.

In some embodiments, the raised letter or shape of the pad is selected from a raised "X," "H," "I," rectangle, square, and astroid.

In some embodiments, the catheter-applying step includes holding an extendable button as the applicator.

In some embodiments, the button has a retracted state and an extended state. The button in the retracted state is recessed into or lies against the catheter back. The button in the extended state is configured to accept a pair of fingers under the button for the holding of the button.

In some embodiments, the method further includes another pump-switching step, a catheter-exchanging step, and a repeating step. The other pump switching step includes switching off the pump to stop drawing the vacuum. The catheter-exchanging step includes exchanging the FEC for a fresh FEC as needed. The repeating step includes repeating for the fresh FEC the catheter-connecting step, the catheter-applying step, the pump-switching step of the switching on of the pump, and the urine-voiding step.

These and other features of the concepts provided herein will become more apparent to those of skill in the art in view of the accompanying drawings and following description, which describe particular embodiments of such concepts in greater detail.

### DRAWINGS

FIG. 1 illustrates a urine-drainage system including an FEC in accordance with some embodiments.
FIG. 2 illustrates the FEC with a first applicator in accordance with some embodiments.
FIG. 3 illustrates the FEC with a second applicator in accordance with some embodiments.
FIG. 4 illustrates the FEC with a third applicator in accordance with some embodiments.
FIG. 5 illustrates the FEC with a fourth applicator in accordance with some embodiments.
FIG. 6 illustrates the FEC with a fifth applicator in accordance with some embodiments.
FIG. 7 illustrates the FEC with a sixth applicator in accordance with some embodiments.
FIG. 8 illustrates the FEC with a seventh applicator in accordance with some embodiments.
FIG. 9A illustrates the FEC with an eighth applicator in a retracted state in accordance with some embodiments.
FIG. 9B illustrates the FEC with the eighth applicator in an extended state in accordance with some embodiments.
FIG. 10 illustrates a patient-facing side of the FEC in accordance with some embodiments.

### DESCRIPTION

Before some particular embodiments are disclosed in greater detail, it should be understood that the particular embodiments disclosed herein do not limit the scope of the concepts provided herein. It should also be understood that a particular embodiment disclosed herein can have features that can be readily separated from the particular embodiment and optionally combined with or substituted for features of any of a number of other embodiments disclosed herein.

Regarding terms used herein, it should also be understood the terms are for the purpose of describing some particular embodiments, and the terms do not limit the scope of the concepts provided herein. Ordinal numbers (e.g., first, second, third, etc.) are generally used to distinguish or identify different features or steps in a group of features or steps, and do not supply a serial or numerical limitation. For example, "first," "second," and "third" features or steps need not necessarily appear in that order, and the particular embodiments including such features or steps need not necessarily be limited to the three features or steps. In addition, any of the foregoing features or steps can, in turn, further include one or more features or steps unless indicated otherwise. Labels such as "left," "right," "top," "bottom," "front," "back," and the like are used for convenience and are not intended to imply, for example, any particular fixed location, orientation, or direction. Instead, such labels are used to reflect, for example, relative location, orientation, or directions. Singular forms of "a," "an," and "the" include plural references unless the context clearly dictates otherwise.

Unless defined otherwise, all technical and scientific terms used herein have the same meaning as commonly understood by those of ordinary skill in the art.

Again, FECs are indicated for non-invasive urine-output management in incontinent female patients. Generally, the FECs operate by drawing urine away from the patients and into urine-drainage systems under vacuum. Intermittent FECs, which are typically used more frequently for shorter periods of time than some other FECs, are most often self-placed by the patients that use them. For this reason, such intermittent FECs should be intuitive and easy to place when needed for voiding urine.

Disclosed herein are applicators for FECs and methods thereof that address at least the foregoing. However, it should be understood that the FECs disclosed herein are not limited to intermittent FECs. Indeed, longer-term FEC such as those configured to be worn for up to 8 hours or more can benefit from the applicators and other FEC features disclosed herein.

### Urine-Drainage Systems

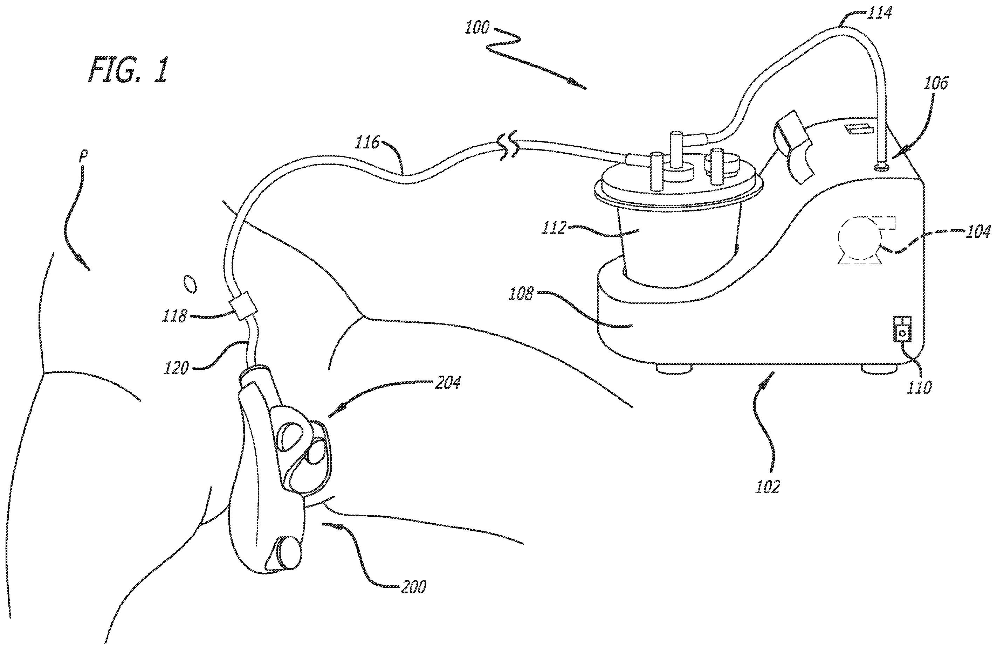

FIG. 1 illustrates a urine-drainage system 100 including an FEC 200 self-placed by a patient P in accordance with some embodiments.

As shown, the urine-drainage system 100 can include a pump unit 102 and the FEC 200 with any applicator of the applicators 204a-204h set forth herein. Notably, the pump unit 102 and its associated equipment (e.g., the collection canister 112, the urine-drainage tubing 116) is multi-use equipment and the FEC 200 is single-use equipment; however, use of some of the multi-use equipment associated with the pump unit 102 can be more limited than that of the pump unit 102 itself as such multi-use equipment might need to be periodically replaced. For example, while the urine-drainage tubing 116 is multi-use equipment, the urine-drainage tubing 116 might need to be periodically replaced.

The pump unit 102 can include an internal pump 104. The pump 104 can be configured to draw a vacuum through an inlet 106 in a housing 108 of the pump unit 102 when a toggle switch 110 is switched on. The pump 104 can also be configured to stop drawing a vacuum through the inlet 106 when the toggle switch 110 is switched off. While not shown, the pump unit 102 can be powered by a general-purpose alternating-current ("AC") electric power supply or one or more batteries.

The multi-use equipment associated with the pump unit 102 can include a urine-collection canister 112, pump tubing 114, and urine-drainage tubing 116. The pump tubing 114 can be configured to fluidly connect the pump 104 to the urine-collection canister 112. The urine-drainage tubing 116 can be configured to fluidly connect the urine-collection canister 112 to the FEC 200 through intervening catheter connector 118 and catheter tubing 120. (Notably, the catheter connector 118 and the catheter tubing 120 are considered part of the FEC 200. Thus, the catheter connector 118 and the catheter tubing 120 are single-use equipment along with the FEC 200.) When such multi-use equipment is fluidly connected to the FEC 200, urine can be drawn from the FEC 200, through the urine-drainage tubing 116, and into the urine-collection canister 112 when the toggle switch 110 is switched on.

### Female external catheters

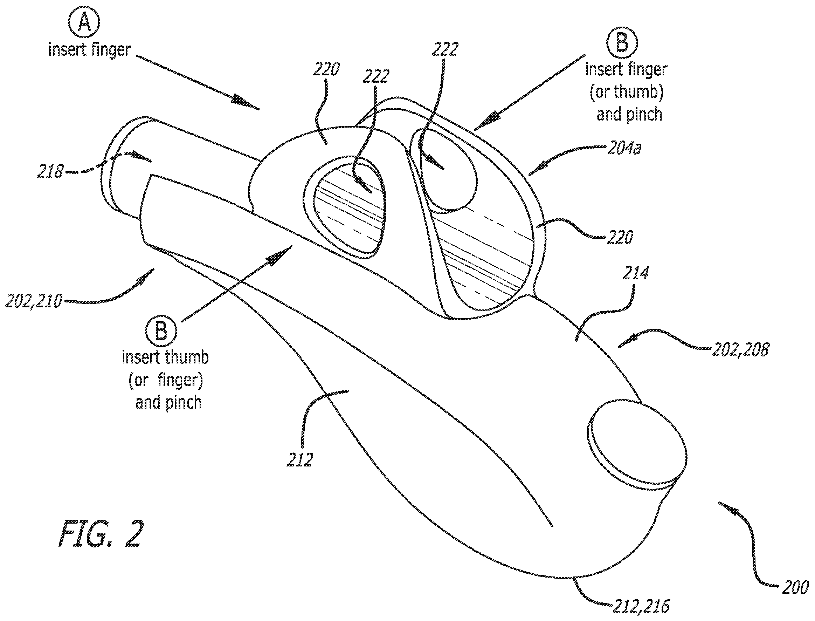

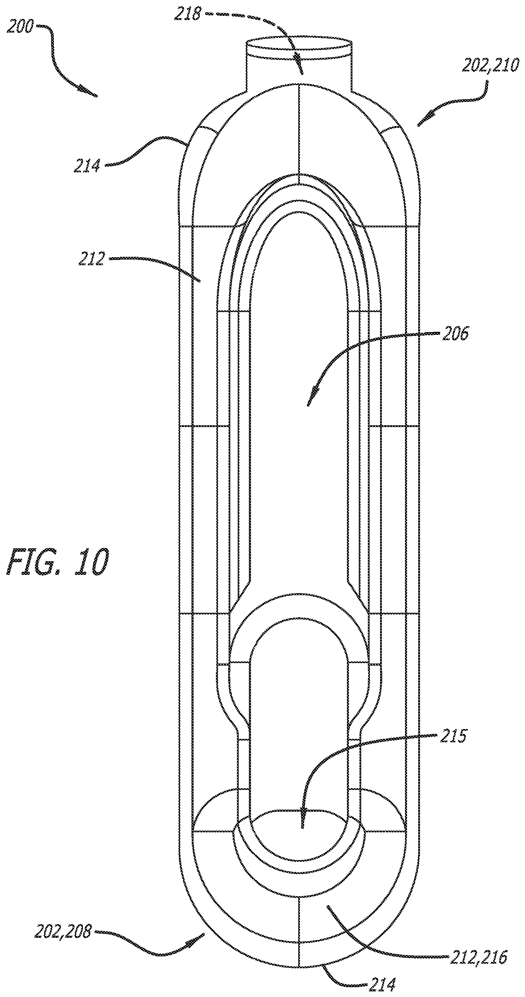

FIGS. 2-10 illustrate the FEC 200 with different applicators in accordance with some embodiments. FIG. 10 illustrates a patient-facing side of the FEC 200 in accordance with some embodiments.

As shown, the FEC 200 can include a body 202 and an applicator 204.

The body 202 can include a cavity 206 extending along a majority of a length of the FEC 200, the length of the FEC 200 being from a sump end 208 to a drainage end 210 of the FEC 200. The body 202 can include a sidewall 212 and a catheter back 214. The body 202 can be integral such that the sidewall 212 and the catheter back 214 are formed together in a single piece, optionally, together with the applicator 204 in a molding process (e.g., compression molding, injection molding, etc.) using, for example, thermoplastic polyurethane ("TPU"). Such an FEC 200 can have a durometer and bulk modulus sufficient to conform to the patient P under pressure via the applicator 204 while remaining comfortable for the patient P.

The cavity 206 can be configured to open toward the patient P for voiding urine into the cavity 206. Indeed, the cavity 206 can include a sump 215 in the sump end 208 of the FEC 200 configured to collect the urine during the voiding of the urine for withdrawal of the urine from the FEC 200.

The sidewall 212 can encircle the cavity 206. Notably, the sidewall 212 can include a knoll 216 in the sump end 208 of the FEC 200 that extends over the cavity 206 and the sump 215 thereof. The knoll 216 can be configured to be disposed over a vaginal introitus of the patient P when the FEC 200 is tucked between inner labia of the patient P.

The catheter back 214 can be opposite the cavity 206, and, like the cavity 206, the catheter back 214 can extend along the majority of the length of the FEC 200. The catheter back 214 can include a conduit 218 extending along the length of the FEC 200 from the sump 215 through the drainage end 210 of the FEC 200. Again, the body 202 can be integral such that the sidewall 212 and the catheter back 214 are formed together in a single piece in a molding process. Notably, the conduit 218, too, can be formed in such a molding process. The catheter tubing 120 can be disposed in a portion of the conduit 218 up to an entirety of the conduit 218 for withdrawal of the urine from the FEC 200.

The applicator 204 can be over the catheter back 214. The applicator 204 can be configured for self-applying the FEC 200 and self-holding the FEC 200 in place during the voiding of the urine into the cavity 206. Again, the applicator 204 can be formed in a molding process together with the body 202. However, the applicator 204 such as the applicator 204h can be formed separately and disposed in the catheter back 214 as needed or desired. Notably, the "applicator 204" is a reference to the genus of applicators provided herein, whereas the "applicator 204a," the "applicator 204b," the "applicator 204c," etc. are references to different species of applicators provided herein.

FIG. 2 illustrates the FEC 200 with a first applicator 204a in accordance with some embodiments.

As shown, the applicator 204a is a finger clip over the catheter back 214. The finger-clip applicator 204a can include a pair of arcuate clip arms 220 extending along at least a portion of the length of the FEC 200. The pair of clip arms 220 can open toward each other to form a cradle configured to accept a finger slidably disposed in the finger-clip applicator 204a from the drainage end 210 of the FEC 200. Each clip arm of the pair of clip arms 220 can include a finger through hole 222 therethrough providing a pair of finger through holes 222. When present, the pair of finger through holes 222 can be configured to accept a finger and a thumb therein for pinching and holding the pair of clip arms 220.

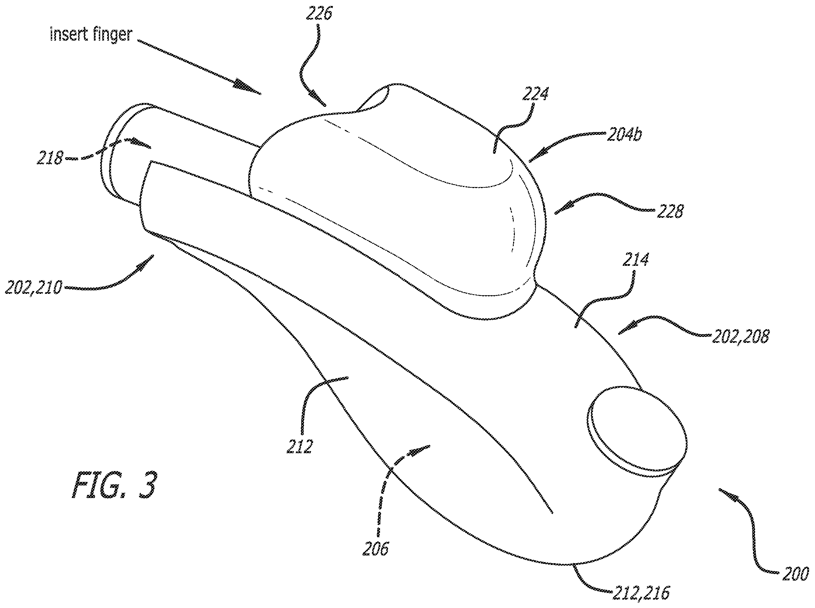

FIG. 3 illustrates the FEC 200 with a second applicator 204b in accordance with some embodiments.

As shown, the applicator 204b is a finger pocket over the catheter back 214. The finger-pocket applicator 204b can include a tubular body 224 extending along at least a portion of the length of the FEC 200. The tubular body 224 can include an open end 226 opening toward the drainage end 210 of the FEC 200 and a closed end 228 opposite the open end 226. The finger-pocket applicator 204b can be configured to accept a finger slidably disposed in the finger-pocket applicator 204b from the open end 226 of the finger-pocket applicator 204b. Notably, like the finger-clip applicator 204a, the finger-pocket applicator 204b can also include the pair of finger through holes 222, albeit through sides of the finger-pocket applicator 204b. When present, the pair of finger through holes 222 can be configured to accept a finger and a thumb therein for pinching and holding the sides of the finger-pocket applicator 204b.

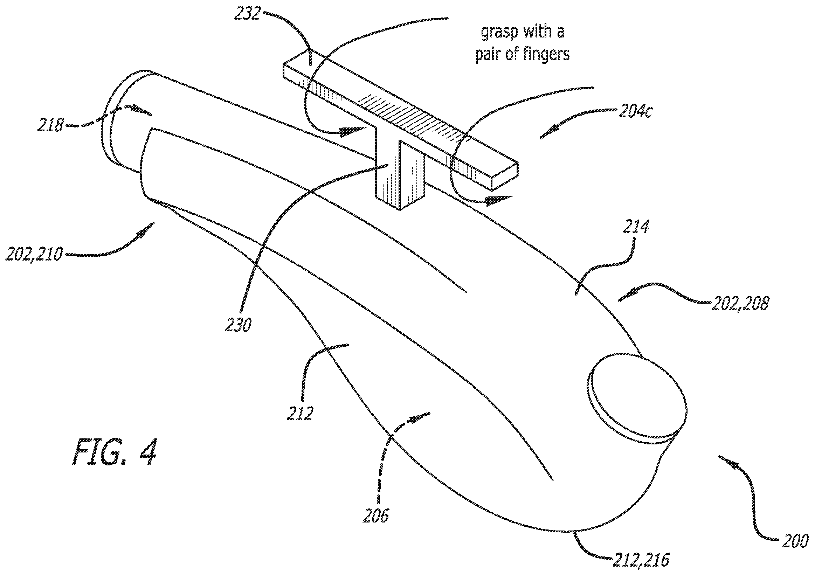

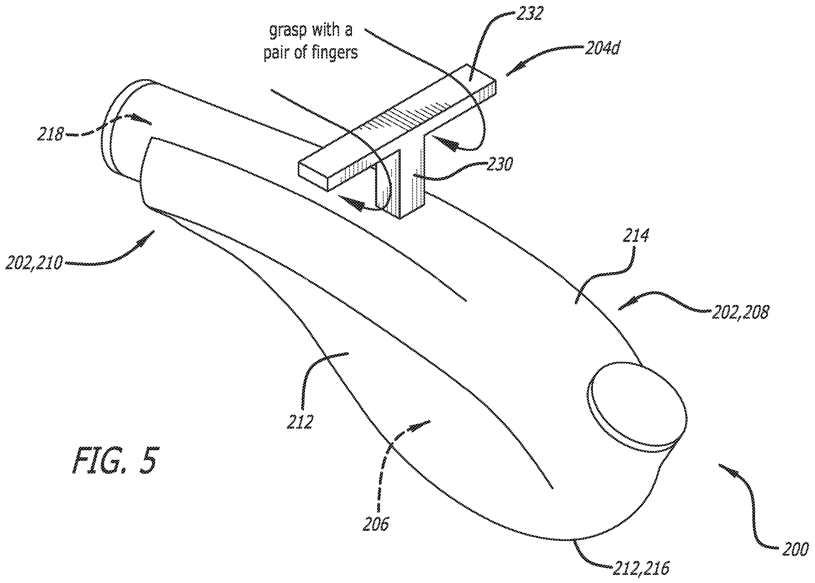

FIG. 4 illustrates the FEC 200 with a third applicator 204c in accordance with some embodiments. FIG. 5 illustrates the FEC 200 with a fourth applicator 204d in accordance with some embodiments.

As shown, each applicator of the applicators 204c and 204d resembles a "T"-shaped handle over the catheter back 214. The handle applicator 204c or 204d can include a post 230 supporting a crossbar 232 that extends along at least a portion of the length of the FEC 200 or orthogonally to the length of the FEC 200. That is, the handle applicator 204d can extend along at least a width of the FEC 200. The handle applicator 204c or 204d can be configured to accept a pair of fingers slidably disposed under the crossbar 232 for holding the handle applicator 204c or 204d.

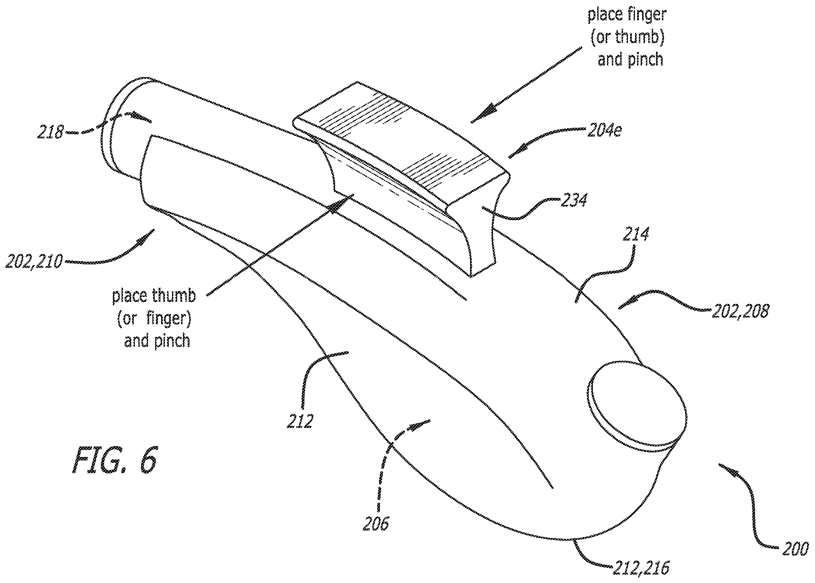

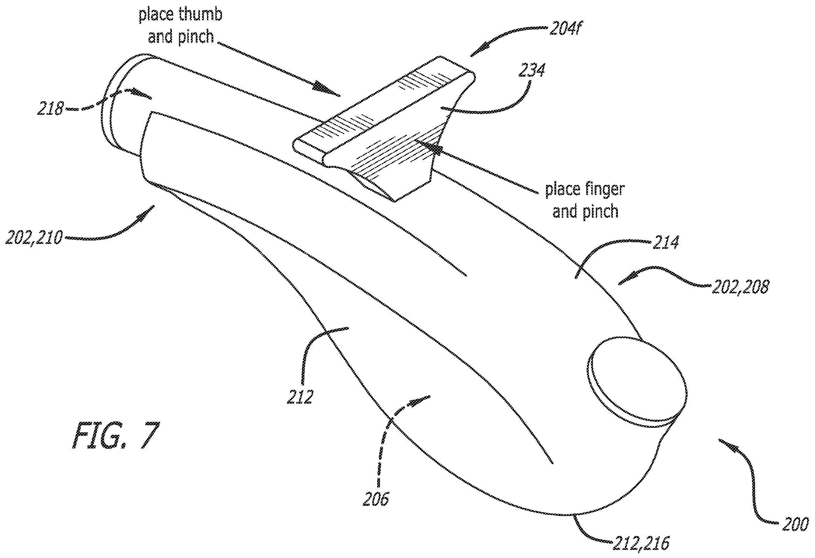

FIG. 6 illustrates the FEC 200 with a fifth applicator 204e in accordance with some embodiments. FIG. 7 illustrates the FEC 200 with a sixth applicator 204f in accordance with some embodiments.

As shown, the applicator 204e r 204f is a pinchable ridge 234 over the catheter back 214. The ridge applicator 204e can extend along at least a portion of the length of the FEC 200, whereas the ridge applicator 204f can extend orthogonally to the length of the FEC 200. That is, the ridge applicator 204f can extend along at least the width of the FEC 200. The ridge applicator 204e or 204f can be configured to accept a finger and a thumb over the ridge applicator 204e or 204f for pinching and holding the ridge 234. Notably, like the finger-clip applicator 204a, at least the ridge applicator 204e can also include the pair of finger through holes 222, albeit through sides of the ridge applicator 204e. When present, the pair of finger through holes 222 can be configured to accept a finger and a thumb therein for pinching and holding the sides of the ridge applicator 204e.

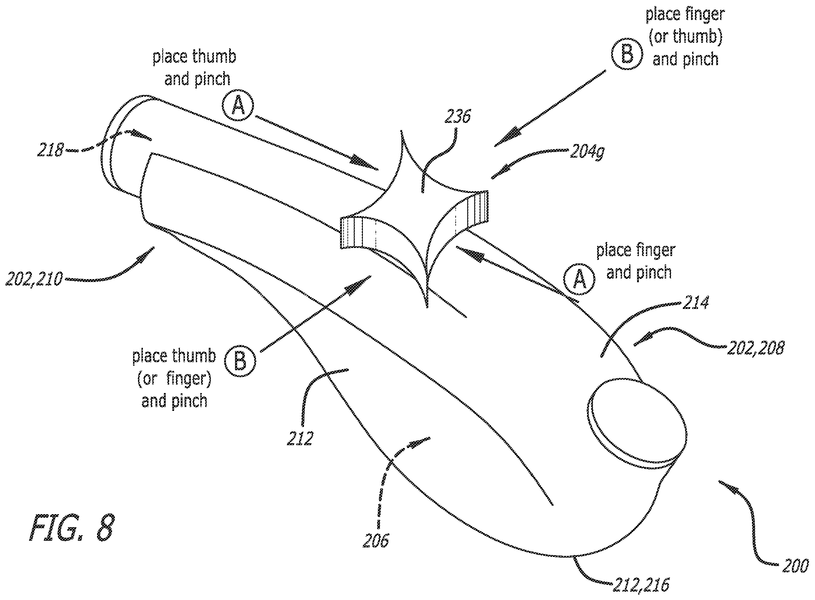

FIG. 8 illustrates the FEC 200 with a seventh applicator 204g in accordance with some embodiments.

As shown, the applicator 204g is a pinchable pad 236 over the catheter back 214. The pad applicator 204g can extend along at least a portion of the length of the FEC 200. The pad applicator 204g can resemble a raised letter or shape configured to accept a finger and a thumb over the pad applicator 204g in either orientation of two orthogonal orientations for pinching and holding the pad applicator 204g. The raised letter of the pad 236 can be selected from a raised "X," "H," "I," or the like. The raised shape of the pad 236 can alternatively be selected from a raised rectangle, square, and astroid. Notably, the pad applicator 204g shown in FIG. 8 resembles a raised astroid.

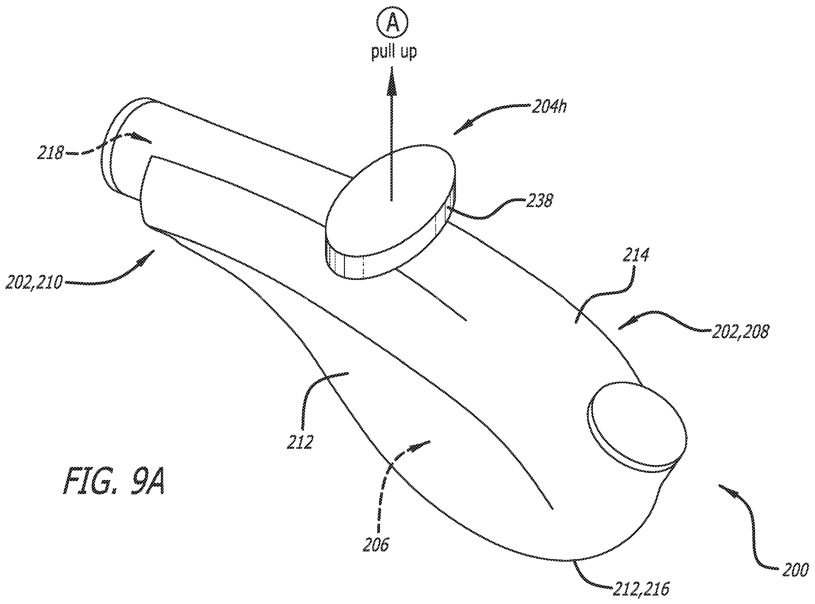

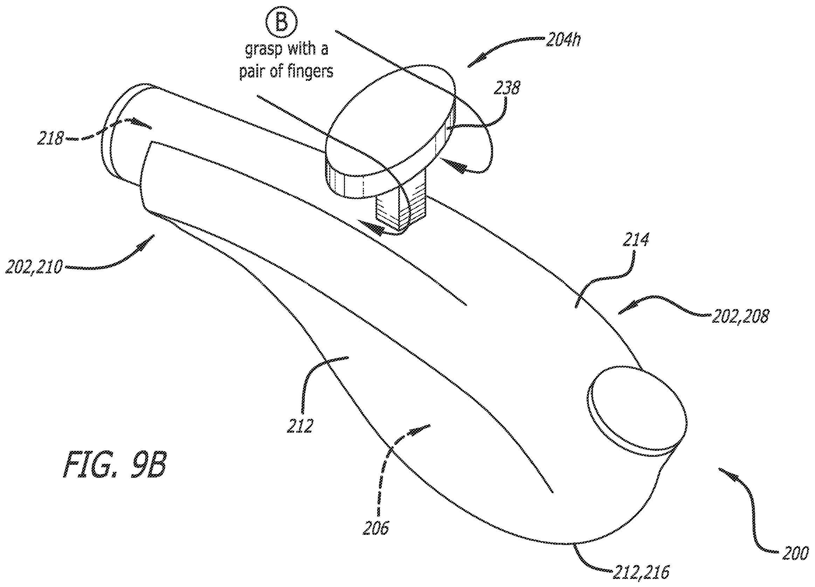

FIG. 9A illustrates the FEC 200 with an eighth applicator 204h in a retracted state in accordance with some embodiments. FIG. 9B illustrates the FEC 200 with the eighth applicator 204h in an extended state in accordance with some embodiments.

As shown, the applicator 204h is an extendable button 238 over the catheter back 214. The button applicator 204h can have a retracted state and an extended state. The button applicator 204h in the retracted state can be recessed into or lie against the catheter back 214. The button applicator 204h in the extended state can be configured to accept a pair of fingers slidably disposed under the button applicator 204h for holding the button 238.

Notably, any embodiment of the applicator 204 set forth above can be ergonomically modified to accommodate left- or right-handed patients. For example, instead of the finger-clip applicator 204a, the finger-pocket applicator 204b, the handle applicator 204c or 204d, or the ridge applicator 204e extending along the length of the FEC 200 in-line with the length of the FEC 200 as shown in FIGS. 2, 3, 4, and 6, respectively, the finger-clip applicator 204a, the finger-pocket applicator 204b, the handle applicator 204c or 204d, or the ridge applicator 204e can extend obliquely along the length of the FEC 200 to accommodate the left- or right-handed patients. While imparting a left- or right-handedness to the FEC 200 in accordance with the foregoing might require manufacturing at least two mirror-imaged FECs, such handed FECs can advantageously reduce repetitive-strain injuries in long term-using patients.

### Methods

Methods of the FEC 200 include methods of using the FEC 200. For example, a method of using the FEC 200 can include at least one or more steps selected a catheter-connecting step, a catheter-applying step, a pump-switching step, and a urine-voiding step.

The catheter-connecting step can include connecting the catheter tubing 120 of the FEC 200 to the urine-drainage tubing 116 of a remainder of the urine-drainage system 100.

The catheter-applying step can include applying the FEC 200 such that the knoll 216 is disposed over the vaginal introitus of the patient P when the FEC 200 is tucked between inner labia of the patient P. The applying of the FEC 200 can utilize the applicator 204 over the catheter back 214 of the FEC 200 opposite the cavity 206. Indeed, the catheter-applying step can include inserting a finger into the finger pocket of the finger-pocket applicator 204a, pinching or inserting a same or different finger into the finger clip of the finger-clip applicator 204b, holding the handle of the handle applicator 204c or 204d, pinching the ridge 234 of the ridge applicator 204e or 204f, pinching the pad 236 of the pad applicator 204g, or holding the extendable button 238 of the button applicator 204h.

The pump-switching step can include switching on the pump 104 of the pump unit 102 of the urine-drainage system 100 to draw a vacuum through the cavity 206.

The urine-voiding step can include voiding urine into the FEC 200 such that the urine is drawn into the cavity 206, through the sump 215 in the sump end 208 of the FEC 200, and out the drainage end 210 of the FEC 200 opposite the sump end 208 of the FEC 200 by way of the catheter tubing 120.

Notably, the method can further include another pump-switching step, a catheter-exchanging step, and a repeating step. The other pump switching step can include switching off the pump 104 to stop drawing the vacuum. The catheter-exchanging step can include exchanging the FEC 200 for a fresh FEC as needed. The repeating step can include repeating for the fresh FEC the catheter-connecting step, the catheter-applying step, the pump-switching step of the switching on of the pump 104, and the urine-voiding step. The other pump switching step and the catheter-exchanging step can likewise be repeated as needed.

While some particular embodiments have been disclosed herein, and while the particular embodiments have been disclosed in some detail, it is not the intention for the particular embodiments to limit the scope of the concepts provided herein. Additional adaptations or modifications can appear to those of ordinary skill in the art, and, in broader aspects, these adaptations or modifications are encompassed as well. Accordingly, departures may be made from the particular embodiments disclosed herein without departing from the scope of the concepts provided herein.

## Claims

1. A female external catheter ("FEC") (200), comprising:
a body (202) including:
a cavity (206) extending along a majority of a length of the FEC (200), the length of the FEC (200) being from a sump end (208) to a drainage end (210) of the FEC (200);
a sidewall (212) encircling the cavity (206), the cavity (206) configured to open toward a patient for voiding urine therein;
a catheter back (214) opposite the cavity (206), the catheter back (214) extending from the sump end (208) of the FEC (200) to the drainage end (210) of the FEC (200) opposite the sump end (208) of the FEC (200), wherein the sidewall (212) and the catheter back (214) form an integral body of the FEC (200); and
an applicator (204) over the catheter back (214) configured for applying the FEC (200) and holding the FEC (200) in place during the voiding of the urine,
**characterized in that** said body (202) further includes a knoll (216) of the sidewall (212) extending over the cavity (206) in the sump end (208) of the FEC (200); the knoll (216) configured to be disposed over a vaginal introitus of the patient when the FEC (200) is tucked between inner labia of the patient.

2. The FEC (200) of claim 1, wherein the applicator (204) is a finger pocket over the catheter back (214),
preferably wherein the finger pocket includes a tubular body (224) extending along at least a portion of the length of the FEC (200) with an open end (226) opening toward the drainage end (210) of the FEC (200) and a closed end (228) opposite the open end (226), the finger pocket configured to accept a finger slidably disposed therein from the open end (226) of the finger pocket.

3. The FEC (200) of claim 1, wherein the applicator (204) is a finger clip over the catheter back (214).

4. The FEC (200) of claim 3, wherein the finger clip includes a pair of arcuate clip arms (220) extending along at least a portion of the length of the FEC (200), the pair of clip arms (220) opening toward each other to form a cradle configured to accept a finger slidably disposed therein from the drainage end (210) of the FEC (200).

5. The FEC (200) of claim 4, wherein each clip arm (220) of the pair of clip arms (220) includes a finger through hole (222) therethrough providing a pair of finger through holes (222), the pair of finger through holes (222) configured to accept a finger and a thumb therein for pinching and holding the pair of arcuate clip arms (220).

6. The FEC (200) of claim 1, wherein the applicator (204) resembles a "T"-shaped handle over the catheter back (214),
preferably wherein the handle includes a post (230) supporting a crossbar (232) that extends along at least a portion of the length of the FEC (200) or orthogonally thereto, the handle configured to accept a pair of fingers slidably disposed under the crossbar for holding the handle.

7. The FEC (200) of claim 1, wherein the applicator (204) is a pinchable ridge (234) over the catheter back (214),
preferably wherein the ridge (234) extends along at least a portion of the length of the FEC (200) or orthogonally thereto, the ridge (234) configured to accept a finger and a thumb thereover for pinching and holding the ridge (234).

8. The FEC (200) of claim 1, wherein the applicator (204) is a pinchable pad (236) over the catheter back (214).

9. The FEC (200) of claim 8, wherein the pad (236) extends along at least a portion of the length of the FEC (200), the pad (236) resembling a raised letter or shape configured to accept a finger and a thumb thereover in either orientation of two orthogonal orientations for pinching and holding the pad (236).

10. The FEC (200) of claim 9, wherein the raised letter or shape of the pad (236) is selected from a raised "X," "H," "I," rectangle, square, and astroid.

11. The FEC (200) of claim 1, wherein the applicator (204) is an extendable button (238) over the catheter back (214),
preferably wherein the button (238) has a retracted state and an extended state, the button (238) in the retracted state recessed into or lying against the catheter back (214), and the button (238) in the extended state configured to accept a pair of fingers slidably disposed under the button (238) for holding the button (238).

12. The FEC (200) of any claim of claims 1-11, wherein the sump end (208) of the FEC (200) includes a sump (215) in the cavity (206) configured to collect the urine during the voiding of the urine for withdrawal from the FEC (200).

13. The FEC (200) of claim 12, wherein the catheter back (214) includes a conduit (218) extending along the length of the FEC (200) from the sump (215) through the drainage end (210) of the FEC (200).

14. The FEC (200) of claim 13, further comprising catheter tubing disposed in a portion of the conduit (218) up to an entirety of the conduit (218).

15. The FEC (200) of any claim of claims 1-14, wherein the sidewall (212) and the catheter back (214) form the integral body of the FEC (200) together with the applicator (204).

## Patentansprüche

1. Externer Katheter für Frauen ("FEC") (200), umfassend: einen Körper (202), einschließlich:
einer Kavität (206), die sich entlang eines Großteils der Länge des FEC (200) erstreckt, wobei die Länge des FEC (200) von einem Sammelende (208) bis zu einem Drainageende (210) des FEC (200) reicht;
einer Seitenwand (212), die die Kavität (206) umgibt, wobei die Kavität (206) so konfiguriert ist, dass sie sich zur Patientin hin öffnet, um Urin darin zu entleeren;
einer Katheterrückseite (214) gegenüber der Kavität (206), wobei sich die Katheterrückseite (214) vom Sammelende (208) des FEC (200) zum Drainageende (210) des FEC (200) gegenüber dem Sammelende (208) des FEC (200) erstreckt, wobei die Seitenwand (212) und die Katheterrückseite (214) einen integralen Körper des FEC (200) bilden; und
eines Applikators (204) über der Katheterrückseite (214), konfiguriert zum Anwenden des FEC (200) und zum Halten des FEC (200) an Ort und Stelle während des Urinierens,
**dadurch gekennzeichnet, dass** der Körper (202) weiter eine Erhebung (216) der Seitenwand (212) einschließt, die sich über die Kavität (206) im Sammelende (208) des FEC (200) erstreckt; wobei die Erhebung (216) so konfiguriert ist, dass sie über dem Scheideneingang der Patientin angeordnet ist, wenn der FEC (200) zwischen den inneren Schamlippen der Patientin verstaut ist.

2. FEC (200) nach Anspruch 1, wobei der Applikator (204) eine Fingertasche über der Katheterrückseite (214) ist,
vorzugsweise wobei die Fingertasche einen röhrenförmigen Körper (224) einschließt, der sich entlang mindestens eines Abschnitts der Länge des FEC (200) mit einem offenen Ende (226) erstreckt, das sich zum Drainageende (210) des FEC (200) hin öffnet, und einem verschlossenen Ende (228) gegenüber dem offenen Ende (226), wobei die Fingertasche konfiguriert ist, einen Finger aufzunehmen, der vom offenen Ende (226) der Fingertasche aus gleitend darin angeordnet ist.

3. FEC (200) nach Anspruch 1, wobei der Applikator (204) eine Fingerklemme über der Katheterrückseite (214) ist.

4. FEC (200) nach Anspruch 3, wobei die Fingerklemme ein Paar bogenförmiger Klemmarme (220) einschließt, die sich entlang mindestens eines Abschnitts der Länge des FEC (200) erstrecken, wobei sich das Paar Klemmarme (220) zueinander hin öffnen, um eine Halterung zu bilden, die so konfiguriert ist, dass sie einen Finger aufnehmen kann, der vom Drainageende (210) des FEC (200) aus gleitend darin angeordnet ist.

5. FEC (200) nach Anspruch 4, wobei jeder Klemmarm (220) des Paares von Klemmarmen (220) ein Fingerdurchgangsloch (222) einschließt, das durch ihn hindurch verläuft und ein Paar von Fingerdurchgangslöchern (222) bereitstellt, wobei das Paar von Fingerdurchgangslöchern (222) so konfiguriert ist, dass es einen Finger und einen Daumen aufnehmen kann, um das Paar von bogenförmigen Klemmarmen (220) zusammenzudrücken und zu halten.

6. FEC (200) nach Anspruch 1, wobei der Applikator (204) einem T-förmigen Griff über der Katheterrückseite (214) ähnelt,
wobei der Griff vorzugsweise einen Pfosten (230) einschließt, der eine Querstange (232) trägt, die sich entlang mindestens eines Abschnitts der Länge des FEC (200) oder orthogonal dazu erstreckt, wobei der Griff so konfiguriert ist, dass er ein Paar Finger aufnehmen kann, die unter der Querstange verschiebbar angeordnet sind, um den Griff zu halten.

7. FEC (200) nach Anspruch 1, wobei der Applikator (204) eine zusammendrückbare Rippe (234) über der Katheterrückseite (214) ist,
vorzugsweise wobei sich die Rippe (234) entlang mindestens eines Abschnitts der Länge des FEC (200) oder orthogonal dazu erstreckt, wobei die Rippe (234) so konfiguriert ist, dass sie einen Finger und einen Daumen zum Zusammendrücken und Halten der Rippe (234) entlang mindestens eines Abschnitts der Länge aufnehmen kann.

8. FEC (200) nach Anspruch 1, wobei der Applikator (204) ein zusammendrückbares Pad (236) über der Katheterrückseite (214) ist.

9. FEC (200) nach Anspruch 8, wobei sich das Polster (236) entlang mindestens eines Abschnitts der Länge des FEC (200) erstreckt, wobei das Polster (236) einem erhabenen Buchstaben oder einer erhabenen Form ähnelt, das so konfiguriert ist, dass es einen Finger und einen Daumen zum Zusammendrücken und Halten des Pads (236) in beiden orthogonalen Ausrichtungen aufnehmen kann.

10. FEC (200) nach Anspruch 9, wobei der erhabene Buchstabe oder die erhabene Form des Pads (236) aus einem erhabenen "X", "H", "I", Rechteck, Quadrat und Astroid ausgewählt wird.

11. FEC (200) nach Anspruch 1, wobei der Applikator (204) ein erweiterbarer Knopf (238) über der Katheterrückseite (214) ist,
vorzugsweise wobei der Knopf (238) einen zurückgezogenen Zustand und einen ausgefahrenen Zustand aufweist, wobei der Knopf (238) im zurückgezogenen Zustand in der Katheterrückseite (214) versenkt ist oder an dieser anliegt, und der Knopf (238) im ausgefahrenen Zustand so konfiguriert ist, dass er ein Paar Finger aufnehmen kann, die unter dem Knopf (238) verschiebbar angeordnet sind, um den Knopf (238) zu halten.

12. FEC (200) nach einem der Ansprüche 1-11, wobei das Sammelende (208) des FEC (200) einen Sammelbehälter (215) in der Kavität (206) einschließt, der so konfiguriert ist, dass er den Urin während des Urinierens sammelt, um ihn aus dem FEC (200) zurückziehen zu können.

13. FEC (200) nach Anspruch 12, wobei die Katheterrückseite (214) eine Leitung (218) einschließt, die sich entlang der Länge des FEC (200) vom Sammelbehälter (215) bis zum Drainageende (210) des FEC (200) erstreckt.

14. FEC (200) nach Anspruch 13, weiter umfassend einen Katheterschlauch, der in einem Abschnitt der Leitung (218) bis hin zur gesamten Leitung (218) angeordnet ist.

15. FEC (200) nach einem der Ansprüche 1-14, wobei die Seitenwand (212) und die Katheterrückseite (214) zusammen mit dem Applikator (204) den integralen Körper des FEC (200) bilden.

## Revendications

1. Cathéter externe féminin (« FEC ») (200), comprenant : un corps (202) incluant :
une cavité (206), s'étendant sur la majeure partie d'une longueur du FEC (200), la longueur du FEC (200) allant d'une extrémité (208) de puisard à une extrémité (210) de drainage du FEC (200) ;
une paroi latérale (212), entourant la cavité (206), la cavité (206) étant conçue pour s'ouvrir vers une patiente afin d'évacuer de l'urine dans celle-ci ;
un dos (214) de cathéter opposé à la cavité (206), le dos (214) de cathéter s'étendant de l'extrémité (208) de puisard du FEC (200) à l'extrémité (210) de drainage du FEC (200) opposée à l'extrémité (208) de puisard du FEC (200), la paroi latérale (212) et le dos (214) de cathéter formant un corps du FEC (200) en un seul tenant ; et
un applicateur (204) sur le dos (214) de cathéter, conçu pour appliquer le FEC (200) et maintenir le FEC (200) en place pendant l'évacuation de l'urine,
**caractérisé en ce que** ledit corps (202) inclut en outre une butte (216) de la paroi latérale (212) s'étendant sur la cavité (206) dans l'extrémité (208) de puisard du FEC (200) ; la butte (216) est conçue de façon à être disposée sur un introïtus vaginal de la patiente lorsque le FEC (200) est placé entre les lèvres internes de la patiente.

2. FEC (200) selon la revendication 1, dans lequel l'applicateur (204) est une poche pour doigt sur le dos (214) de cathéter, de préférence dans lequel la poche pour doigt inclut un corps tubulaire (224) s'étendant le long d'au moins une partie de la longueur du FEC (200) avec une extrémité ouverte (226) s'ouvrant vers l'extrémité (210) de drainage du FEC (200) et une extrémité fermée (228) opposée à l'extrémité ouverte (226), la poche pour doigt étant conçue pour accueillir un doigt disposé de manière à pouvoir glisser dans celle-ci à partir de l'extrémité ouverte (226) de la poche pour doigt.

3. FEC (200) selon la revendication 1, dans lequel l'applicateur (204) est une pince à doigt sur le dos (214) de cathéter.

4. FEC (200) selon la revendication 3, dans lequel la pince à doigt inclut une paire de bras (220) de pince arqués s'étendant le long d'au moins une partie de la longueur du FEC (200), la paire de bras (220) de pince s'ouvrant l'un vers l'autre pour former un berceau conçu pour accueillir un doigt disposé de manière à pouvoir glisser dans celui-ci à partir de l'extrémité (210) de drainage du FEC (200).

5. FEC (200) selon la revendication 4, dans lequel chaque bras (220) de pince de la paire de bras (220) de pince inclut un trou traversant (222) pour doigt à travers celui-ci fournissant une paire de trous traversants (222) pour doigt, la paire de trous traversants (222) pour doigt étant conçue pour accueillir un doigt et un pouce à l'intérieur de ceux-ci pour pincer et maintenir la paire de bras (220) de pince arqués.

6. FEC (200) selon la revendication 1, dans lequel l'applicateur (204) ressemble à une poignée en forme de « T » sur le dos (214) de cathéter, de préférence dans lequel la poignée inclut un montant (230) supportant une barre transversale (232) qui s'étend le long d'au moins une partie de la longueur du FEC (200) ou orthogonalement à celle-ci, la poignée étant conçue pour accueillir une paire de doigts disposés de manière à pouvoir glisser sous la barre transversale pour maintenir la poignée.

7. FEC (200) selon la revendication 1, dans lequel l'applicateur (204) est une crête (234) pouvant être pincée sur le dos (214) de cathéter, de préférence dans lequel la crête (234) s'étend le long d'au moins une partie de la longueur du FEC (200) ou orthogonalement à celle-ci, la crête (234) étant conçue pour accueillir un doigt et un pouce sur celle-ci pour pincer et maintenir la crête (234).

8. FEC (200) selon la revendication 1, dans lequel l'applicateur (204) est un patin (236) pouvant être pincé sur le dos (214) de cathéter.

9. FEC (200) selon la revendication 8, dans lequel le patin (236) s'étend le long d'au moins une partie de la longueur du FEC (200), le patin (236) ressemblant à une lettre ou forme en relief conçue pour accueillir un doigt et un pouce sur celle-ci dans l'une ou l'autre parmi deux orientations orthogonales pour pincer et maintenir le patin (236).

10. **FEC (200)** selon la revendication 9, dans lequel la lettre ou forme en relief du patin (236) est choisie parmi un « X », un « H », un « I », un rectangle, un carré et un astroïde en relief.

11. FEC (200) selon la revendication 1, dans lequel l'applicateur (204) est un bouton extensible (238) sur le dos (214) de cathéter, de préférence dans lequel le bouton (238) présente un état rétracté et un état étendu, le bouton (238) dans l'état rétracté étant encastré dans le dos (214) de cathéter ou reposant contre celui-ci, et le bouton (238) dans l'état étendu étant conçu pour accueillir une paire de doigts disposés de manière à pouvoir glisser sous le bouton (238) pour maintenir le bouton (238).

12. FEC (200) selon une quelconque revendication des revendications 1 à 11 dans lequel l'extrémité (208) de puisard du FEC (200) inclut un puisard (215) dans la cavité (206) conçu pour recueillir l'urine lors de l'évacuation de l'urine pour le retrait du FEC (200).

13. FEC (200) selon la revendication 12, dans lequel le dos (214) de cathéter inclut un conduit (218) s'étendant le long de la longueur du FEC (200) depuis le puisard (215) jusqu'à l'extrémité (210) de drainage du FEC (200).

14. FEC (200) selon la revendication 13, comprenant en outre une tubulure de cathéter disposée dans une partie du conduit (218) jusqu'à la totalité du conduit (218).

15. FEC (200) selon une quelconque revendication des revendications 1 à 14, dans lequel la paroi latérale (212) et le dos (214) de cathéter forment le corps intégral du FEC (200) avec l'applicateur (204).
